# EUROPÄISCHE PATENTANMELDUNG

(11) **EP 1 642 891 A1**
(43) Veröffentlichungstag der Anmeldung: **05.04.2006**
(21) Anmeldenummer: 05020328.0
(22) Anmeldetag: 17.09.2005
(51) Int. Cl.: C07D 207/28

(54) **Verfahren zur enzymatischen Synthese von Pyroglutaminsäureestern**

(30) Priorität: 01.10.2004 DE 102004047869
(71) Anmelder: Goldschmidt GmbH, 45127 Essen (DE)
(72) Erfinder: Pascaly, Matthias, Dr., 48163 Münster (DE); Thum, Oliver, Dr., 40880 Ratingen (DE)

(57) **Zusammenfassung**

Gegenstand der vorliegenden Erfindung ist ein Verfahren zur Herstellung von Verbindungen gemäß der allgemeinen Formel (I), 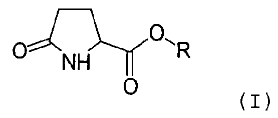 worin R der Kohlenwasserstoffrest eines Alkohols ist, welcher dadurch gekennzeichnet ist, dass ein oder mehrere Alkohole der allgemeinen Formel (II)

R-OH (II)

worin R wie oben definiert ist, ohne Verwendung wesentlicher Mengen, vorzugsweise ohne Lösungsmittel, vorzugsweise unter vermindertem Druck bei Temperaturen unterhalb 100 °C in Gegenwart von Enzymen aus der Klasse der Hydrolasen mit einer Verbindung gemäß der allgemeinen Formel (III), 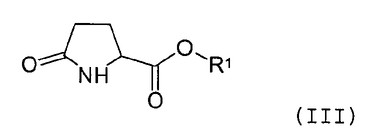 worin R¹ entweder Wasserstoff oder eine lineare oder verzweigte Alkyl- oder Alkenylgruppe mit 1 bis 3 Kohlenstoffatomen ist, ver- und/oder umgeestert werden.

## Beschreibung

Die Erfindung betrifft ein enzymatisches Verfahren zur Herstellung von Pyroglutaminsäureestern.

Es ist bekannt, dass Pyroglutaminsäure (2-Pyrrolidon-5-carbonsäure) eine feuchtigkeitsregulierende Funktion hat, indem sie den Wassergehalt des Stratum Corneum der Haut auch bei niedriger Luftfeuchtigkeit erhöht. Das Natriumsalz ist als wasserlösliche hygroskopische Substanz Bestandteil des natürlichen Feuchthalte-Faktors (natural moisturizing factor, NMF; Gesslein in: Conditioning Agents for Hair and Skin, Seite 95-110; Eds.: R. Schueller, P. Romanowski; Marcel Dekker; New York/Basel 1999). Es ist einer der besten bekannten Moisturizer für Personal Care Anwendungen.

Pyroglutaminsäure oder deren Salze, die beide auf Grund ihrer Hydrophilie bei lokaler Anwendung nur eine begrenzte Eindringbarkeit in Haut und Haar besitzen, werden schnell durch Wasser weggewaschen, wodurch langfristige Effekte, wie ein nachhaltiges Feuchtigkeitsspenden, verhindert werden.

Daher werden Alkylester der Pyroglutaminsäure in kosmetischen Formulierungen genutzt, um der Haut Feuchtigkeit zu spenden oder gar das Haarwachstum zu beschleunigen (EP-B-0 342 054, EP-B-0 342 055, EP-B-0 342 056). Diese Eigenschaft beruht auf der Fähigkeit dieser Verbindungen, durch die Haut in das Stratum Corneum zu penetrieren, wo diese Verbindungen durch die dort anwesende Pyroglutamatpeptidase (EC 3.4.19.3; J. Invest. Dermatol. 1983, 81, 122-127) unter Freisetzung der freien Pyroglutaminsäure gespalten werden.

Dem häufigen Einsatz in kosmetischen Anwendungen stand bisher das Fehlen einfacher und kostengünstiger Herstellmethoden entgegen. Der gegenwärtige Stand der Technik setzt konventionelle Veresterungskatalysatoren, wie z. B. Schwefelsäure, Thionylchlorid und Chlorwasserstoff, ein, um unter recht drastischen Bedingungen und unter Verwendung von Lösungsmitteln die gewünschten Produkte zu gewinnen (Beispiele siehe DE-2102172 und DE-210217). In neuerer Zeit wurde auch die alternative Synthese unter Verwendung von Mikrowellenöfen beschrieben (FR-A-2 833 260).

Kürzlich wurde auch die biokatalytische Darstellung von Pyroglutaminsäureestern durch enzymatische Umesterung beschrieben (Enzyme Microbial Technol. 2003, 33, 79-83; Biotechnol. Lett. 2004, 26, 193-196). Allerdings erfüllt auch dieses Verfahren nicht die Ansprüche, die nötig sind, um eine industrielle Anwendung zu ermöglichen.

Zunächst beruht dieses Verfahren auf dem Einsatz organischer Lösungsmittel, was zum einen für die Verwendung in kosmetischen Formulierungen absolut unerwünscht ist und zum anderen zusätzliche Herstellkosten verursacht.

Weiterhin müssen selbst zum Erreichen der beschriebenen Umsätze (üblicherweise 65 bis 73 %) große Überschüsse der Alkohole (üblicherweise 5 Äquivalente) und große Mengen Enzym (bis zu 10 Gew.-%) eingesetzt werden.

Die so erhaltene Reaktionsmischung kann dann erst nach umfangreichen Aufarbeitungsschritten für kosmetische Anwendungen eingesetzt werden:
So muss zunächst das organische Lösungsmittel und der überschüssige Alkohol destillativ entfernt werden. Bei längerkettigen Fettalkohlen sind trotz niedriger Drücke oft noch hohe Temperaturen notwendig, wodurch ein Vorteil des enzymatischen Verfahrens, nämlich die Umsetzung unter schonenden Bedingungen, wieder zunichte gemacht wird.
Schließlich ist dieses Verfahren darauf beschränkt, reine Pyroglutaminsäureethyl- oder -methylester als Ausgangsverbindung einzusetzen, d.h. dass diese Ester zunächst aufwändig in reiner Form dargestellt werden müssen.

In Anbetracht des großen Potentials der Pyroglutaminsäureester und den erheblichen Nachteilen der gegenwärtig verfügbaren Produktionsmethoden besteht daher weiterhin ein reger Bedarf an einem Verfahren, mit dem diese Produkte schnell und in nahezu quantitativen Ausbeuten kostengünstig hergestellt werden können, ausgehend von niedermolekularen Pyroglutaminsäurederivaten technischer Qualität.

Eine Aufgabe der vorliegenden Erfindung bestand daher darin, ein Verfahren zu entwickeln, welches eben diesen Ansprüchen genügt.

Überraschenderweise wurde gefunden, dass Pyroglutaminsäure und deren Ester von kurzkettigen Alkoholen enzymatisch mit Alkoholen in einem technisch einfachen Verfahren ver- bzw. umgeestert werden können (siehe Schema 1)

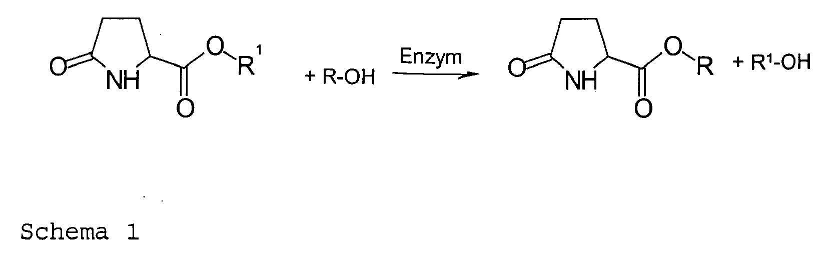

Gegenstand der vorliegenden Erfindung ist daher ein Verfahren zur Herstellung von Verbindungen gemäß der allgemeinen Formel (I), 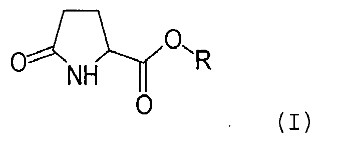 worin R der Kohlenwasserstoffrest eines Alkohols mit ≥ 4 C-Atomen ist, welcher dadurch gekennzeichnet ist, dass ein oder mehrere Alkohole der allgemeinen Formel (II)

R-OH (II)

worin R wie oben definiert ist, ohne Verwendung wesentlicher Mengen Lösungsmittel, vorzugsweise ohne Lösungsmittel, vorzugsweise unter vermindertem Druck bei Temperaturen unterhalb 100 °C in Gegenwart von Enzymen, vorzugsweise von Enzymen aus der Klasse der hydrolytischen Enzyme mit einer Verbindung gemäß der allgemeinen Formel (III) , 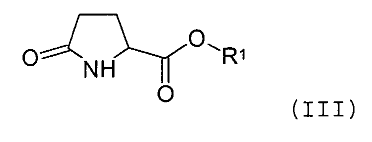 worin R¹ entweder Wasserstoff oder eine lineare oder verzweigte Alkyl- oder Alkenylgruppe mit 1 bis 3 Kohlenstoffatomen ist, ver- und/oder umgeestert werden.

Die erfindungsgemäß verwendeten Verbindungen der allgemeinen Formel (III) sind sowohl Pyroglutaminsäure (R¹ = H), deren Ester mit kurzkettigen. Alkoholen mit R¹ = Methyl-, Ethyl- , Vinyl oder (i-)Propylrest oder technische Mischungen derselben, wie sie z. B. bei einer unvollständigen Veresterung von Pyroglutaminsäure mit Ethanol oder Methanol resultieren.

Dies können z. B. Mischungen von Verbindungen sein, in denen R¹ ein Wasserstoffatom ist mit solchen, in denen R¹ eine lineare oder verzweigte Alkyl- oder Alkenylgruppe mit 1 bis 3 Kohlenstoffatomen ist, in Verhältnissen, in denen R¹ zu 0 bis 100 Gew.-%, vorzugsweise zu 0 bis 50 Gew.-%, insbesondere zu 10 bis 40 Gew.-% ein Wasserstoffatom ist. Solche Mischungen können bevorzugt erhalten werden, wenn Glutaminsäure mit den entsprechenden kurzkettigen Alkoholen, vorzugsweise Methanol oder Ethanol, unter überkritischen Bedingungen umgesetzt wird, wie beispielsweise in der nicht veröffentlichten Patentanmeldung DE 10 2004 008 042.9 beschrieben.

Die zur Herstellung der erfindungsgemäßen Verbindungen verwendeten Alkohole sind die handelsüblichen Produkte mit 4 bis 22 C-Atomen, wie beispielsweise Butanol, Pentanol, Hexanol, Octanol sowie deren Isomeren wie i-Propanol, i-Butanol, 2-Ethylhexanol, Isononylalkohol.

Weiterhin die Alkohole, die nach bekannten Verfahren aus einbasischen Fettsäuren auf Basis natürlicher pflanzlicher oder tierischer Öle mit 6 bis 22 Kohlenstoffatomen, insbesondere mit 14 bis 18 Kohlenstoffatomen, hergestellt werden, wie Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Eurucasäure, Gadoleinsäure, Rapsölfettsäure, Sojaölfettsäure, Sonnenblumenölfettsäure, Talgölfettsäure, Palmölfettsäure, Palmkernölfettsäure, Kokosfettsäure, welche allein oder in Mischung eingesetzt werden können.

Das Verhältnis der Verbindungen der allgemeinen Formel (II) und der Verbindungen der allgemeinen Formel (III) kann in einem weiten Bereich variiert werden. Dieses Verhältnis sollte vorteilhafterweise kleiner als 2 : 1, vorzugsweise kleiner als 1,5 : 1, insbesondere ca. 1 : 1 betragen.

Bei den erfindungsgemäß verwendbaren Enzymen handelt es sich um solche aus der Gruppe der hydrolytischen Enzyme, z. B. Lipasen, Esterasen oder Proteasen, wie beispielsweise Lipasen aus *Candida rugosa, Pseudomonas sp., Thermomyces langosiosus,* Schweinepankreas, *Mucor miehei, Alcaligines sp.*, Cholesterol Esterase aus *Candida rugosa,* oder Esterase aus Schweineleber, vorzugsweise Lipase B aus *Candida antarctica.*

Die Enzyme können erfindungsgemäß in freier Form oder auf geeignete Träger immobilisiert verwendet werden.

Erfindungsgemäß wird bevorzugt als Enzym immobilisierte Lipase B aus *Candida antarctica* in weniger als 5 Gew.-% (bezogen auf die Einwaage aller Reaktanden), bevorzugt in weniger als 2 Gew.-% eingesetzt.

Bei dem erfindungsgemäßen Verfahren werden die entsprechenden Reaktanden in einem Verhältnis wie oben beschrieben in einem geeigneten Reaktor (z. B. Rundkolben mit Rührer oder in einem Festbettreaktor) gemischt und auf die optimale Arbeitstemperatur des verwendeten Biokatalysators erhitzt. Je nach verwendetem Biokatalysator liegt diese Temperatur bei 20 °C bis 100 °C, bevorzugt bei 35 °C bis 70 °C. Bei Verwendung eines Festbettreaktors wird das Festbett mit dem ausgewählten Enzym befüllt und nach Erreichen der Reaktionstemperatur die Reaktionsmischung durch das Festbett gepumpt. Bei Verzicht auf einen Festbettreaktor wird das Enzym der Reaktionsmischung direkt zugesetzt und nach Beendigung der Reaktion durch geeignete Vorrichtungen abfiltriert.

In dem erfindungsgemäßen Verfahren kann auf den Einsatz von zusätzlichen Lösungsmitteln aller Art gänzlich verzichtet werden. Zum Erreichen möglichst vollständiger Umsätze wird die Reaktion unter vermindertem Druck durchgeführt, wobei das entstehende Reaktionswasser (bei Verwendung von Pyroglutaminsäure), bzw. die frei werdenden kurzkettigen Alkohole (bei Einsatz der entsprechenden Ester) destillativ entfernt werden. Der einzustellende Unterdruck ist abhängig von der Reaktionstemperatur, dem Siedepunkt der gegebenenfalls zu entfernenden Alkohole R¹-OH, bzw. des zu entfernenden Reaktionskondensats. Dieser Druck muss gewährleisten, dass die zu entfernenden Komponenten abdestilliert werden, während der als Edukt verwendete Alkohol gemäß der allgemeinen Formel (II) während der Reaktion möglichst vollständig im Reaktionsgefäß verbleibt.

Bei dem erfindungsgemäßen Verfahren werden in Abhängigkeit der Prozessparameter (Anteil Pyroglutaminsäure als Ausgangsverbindung, Art und Menge des verwendeten Enzyms) binnen 8 bis 24 Stunden quantitative Umsätze erreicht (bestimmt per ¹H-NMR, die Nachweisgrenze dieser Methode liegt bei einem Umsatzgrad von ca. 98 %).

### Beispiele:

### Beispiel 1:

### Herstellung von Pyroglutaminsäureoleylester

In einem Mehrhalsrundkolben werden 19,2 g (129 mmol) einer Mischung von Pyroglutaminsäureethylester und Pyroglutaminsäure (molares Verhältnis 71 : 29) und 34,7 g (129 mmol) Oleylalkohol vorgelegt und auf 60 °C erhitzt. Nach Zugabe von 2,6 g Novozym 435 (immobilisierte Lipase B aus C. *antarctica*) wird Vakuum angelegt (20 mbar) und niedrigsiedende Reaktionsprodukte abdestilliert. Die Reaktionskontrolle erfolgt NMR-spektroskopisch oder durch ein geeignetes chromatographisches Verfahren. Umsatz nach 8 Stunden: 98 %, nach 24 Stunden: > 98 %. Nach Beenden der Reaktion wird das immobilisierte Enzym abfiltriert. Das Filtrat liefert 49,0 g Produkt (100 % der theoretischen Ausbeute) ohne weitere Aufarbeitung als leicht gelbe Flüssigkeit.
¹H-NMR (DMSO-d₆, 400 MHz): δ = 8.0 (br, s, 1H), 5.3 (m, 2H), 4.1 (dd, ³J = 8.6 Hz, 3.9 Hz, 1H), 4.0 (td, ³J = 6.6 Hz, 2.1 Hz, 2H), 2.3 (m, 1H), 2.1 (m, 2H), 2.0 (m, 5H), 1.6 (m, 2H), 1.3 (m, 22H), 0.8 (t, ³J = 6.5 Hz, 3H).

### Beispiel 2:

### Herstellung von Pyroglutaminsäureoleylester

Analog zu Beispiel 1 werden 20 g einer Mischung von Pyroglutaminsäureethylester und Pyroglutaminsäure (molare Verhältnisse siehe Tabelle 1) mit einem Äquivalent Oleylalkohol und 5 Gew.-% Novozym 435 umgesetzt. Nach 8 und 24 Stunden wird der Reaktionsumsatz NMR-spekroskopisch (¹H-NMR in DMSO-d₆) bestimmt.

### Tabelle 1:

NMR-spektroskopisch bestimmter Umsatz der Reaktion verschiedener Mischungen von Pyroglutaminsäure und Pyroglutaminsäureethylester mit Oleylalkohol

| Säure % | Ethylester % | Umsatz 8 h % | Umsatz 24 h % |
|---|---|---|---|
| 0 | 100 | 98 | > 98 |
| 25 | 75 | 98 | > 98 |
| 50 | 50 | 93 | > 98 |
| 75 | 25 | 91 | > 98 |
| 100 | 0 | 91 | > 98 |

### Beispiel 3:

### Herstellung von Pyroglutaminsäureoleylester

In einem Mehrhalsrundkolben werden 19,2 g (129 mmol) einer Mischung von Pyroglutaminsäureethylester und Pyroglutaminsäure (molares Verhältnis 71 : 29) und 34,7 g (129 mmol) Oleylalkohol vorgelegt und auf 60 °C erhitzt. Nach Zugabe von 1,07 g Novozym 435 (immobilisierte Lipase B aus *C. antarctica*) wird Vakuum angelegt (20 mbar) und niedrigsiedende Reaktionsprodukte abdestilliert. Die Reaktionskontrolle erfolgt NMR-spektroskopisch oder durch ein geeignetes chromatographisches Verfahren. Umsatz nach 8 Stunden: 95 %, nach 24 Stunden: > 98 %. Nach Beenden der Reaktion wird das immobilisierte Enzym abfiltriert. Das Filtrat liefert 49,0 g Produkt (100 % der theoretischen Ausbeute) ohne weitere Aufarbeitung als leicht gelbe Flüssigkeit.
¹H-NMR (DMSO-d₆, 400 MHz): δ = 8.0 (br, s, 1H), 5.3 (m, 2H), 4.1 (dd, ³*J* = 8.6 Hz, 3.9 Hz, 1H), 4.0 (td, ³*J* = 6.6 Hz, 2.1 Hz, 2H), 2.3 (m, 1H), 2.1 (m, 2H), 2.0 (m, 5H), 1.6 (m, 2H), 1.3 (m, 22H), 0.8 (t, ³*J* = 6.5 Hz, 3H).

### Beispiel 4:

### Herstellung von Pyroglutaminsäureoctylester

In einem Mehrhalsrundkolben werden 50,0 g (336 mmol) einer Mischung von Pyroglutaminsäureethylester und Pyroglutaminsäure (molares Verhältnis 71 : 29) und 43,7 g (336 mmol) Oktanol vorgelegt und auf 60 °C erhitzt. Nach Zugabe von 4,6 g Novozym 435 (immobilisierte Lipase B aus C. antarctica) wird Vakuum angelegt (20 mbar) und niedrigsiedende Reaktionsprodukte abdestilliert. Die Reaktionskontrolle erfolgt NMR-spektroskopisch oder durch ein geeignetes chromatographisches Verfahren. Umsatz nach 8 Stunden: 95 %, nach 24 Stunden: > 98 %. Nach Beenden der Reaktion wird das immobilisierte Enzym abfiltriert. Das Filtrat liefert 81 g Produkt (100 % der theoretischen Ausbeute) ohne weitere Aufarbeitung als leicht gelbe Flüssigkeit.
¹H-NMR (DMSO-d₆, 400 MHz): δ = 8.0 (br, s, 1H), 4.2 (dd, ³*J* = 8.6 Hz, 4.3 Hz, 1H), 4.1 (t, ³J = 6.5 Hz, 2H), 2.3 (m, 1H), 2.1 (m, 2H), 2.0 (m, 1H), 1.6 (m, 2H), 1.3 (m, 10H), 0.8 (t, ³J = 7.1 Hz, 3H).

### Beispiel 5:

### Herstellung von Pyroglutaminsäurelaurylester

In einem Mehrhalsrundkolben werden 51,0 g (339 mmol) einer Mischung von Pyroglutaminsäureethylester und Pyroglutaminsäure (molares Verhältnis 77 : 23) und 63,1 g (339 mmol) Laurylalkohol vorgelegt und auf 60 °C erhitzt. Nach Zugabe von 5,7 g Novozym 435 (immobilisierte Lipase B aus *C. antarctica*) wird Vakuum angelegt (20 mbar) und niedrigsiedende Reaktionsprodukte abdestilliert. Die Reaktionskontrolle erfolgt NMR-spektroskopisch oder durch ein geeignetes chromatographisches Verfahren. Umsatz nach 8 Stunden: 97 %, nach 24 Stunden: > 98 %. Nach Beenden der Reaktion wird das immobilisierte Enzym abfiltriert. Das Filtrat liefert 100,7 g Produkt (100 % der theoretischen Ausbeute) ohne weitere Aufarbeitung als leicht gelblichen Feststoff.
¹H-NMR (DMSO-d₆, 400 MHz): δ = 8.0 (br, s, 1H), 4.2 (dd, ³*J* = 8.6 Hz, 3.5 Hz, 1H), 4.1 (t, ³*J* = 6.5 Hz, 2H), 2.3 (m, 1H), 2.1 (m, 2H), 2.0 (m, 1H), 1.6 (m, 2H), 1.3 (m, 18H), 0.8 (t, ³*J* = 7.1 Hz, 3H).

### Beispiel 6:

### Herstellung von Pyroglutaminsäure-2-hexyldecylester

In einem Mehrhalsrundkolben werden 27,3 g (177 mmol) einer Mischung von Pyroglutaminsäureethylester und Pyroglutaminsäure (molares Verhältnis 90 : 10) und 42,9 g (177 mmol) 2-Hexyldecylalkohol (Isofol-16) vorgelegt und auf 60 °C erhitzt. Nach Zugabe von 3,4 g Novozym 435 (immobilisierte Lipase B aus *C. antarctica*) wird Vakuum angelegt (20 mbar) und niedrigsiedende Reaktionsprodukte abdestilliert. Die Reaktionskontrolle erfolgt NMR-spektroskopisch oder durch ein geeignetes chromatographisches Verfahren. Umsatz nach 8 Stunden: 97 %, nach 24 Stunden: > 98 %. Nach Beenden der Reaktion wird das immobilisierte Enzym abfiltriert. Das Filtrat liefert 62,5 g Produkt (100 % der theoretischen Ausbeute) ohne weitere Aufarbeitung als leicht gelblichen Feststoff.
¹H-NMR (DMSO-d₆, 400 MHz): δ = 8.0 (br, s, 1H), 4.2 (m, 1H), 4.1 (m, 2H), 2.3 (m, 1H), 2.1 (m, 2H), 1.9 (m, 1H), 1.6 (m, 2H), 1.3 (m, 24H), 0.8 (t, ^{*3*}*J* = 7.1 Hz, 6H).

## Patentansprüche

1. Verfahren zur Herstellung von Verbindungen gemäß der allgemeinen Formel (I), 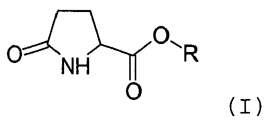 worin R der Kohlenwasserstoffrest eines Alkohols mit ≥ 4 C-Atomen ist, **dadurch gekennzeichnet, dass** ein oder mehrere Alkohole der allgemeinen Formel (II)
R-OH (II)
worin R wie oben definiert ist, ohne Verwendung wesentlicher Mengen Lösungsmittel, vorzugsweise ohne Lösungsmittel, vorzugsweise unter vermindertem Druck bei Temperaturen unterhalb 100 °C in Gegenwart von Enzymen mit einer Verbindung gemäß der allgemeinen Formel (III), 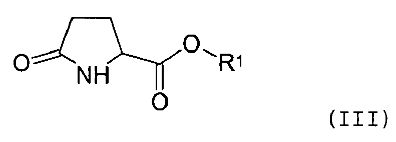 worin R¹ entweder Wasserstoff oder eine lineare oder verzweigte Alkyl- oder Alkenylgruppe mit 1 bis 3 Kohlenstoffatomen ist, ver- und/oder umgeestert werden.

2. Verfahren gemäß Anspruch 1, **dadurch gekennzeichnet, dass** in den Alkoholen der allgemeinen Formel (II) R ein gegebenfalls verzweigter, gegebenenfalls Mehrfachbindungen enthaltender Kohlenwasserstoffrest mit 4 bis 22 C-Atomen ist.

3. Verfahren gemäß mindestens einem der Ansprüche 1 bis 2, **dadurch gekennzeichnet, dass** das Verhältnis der Verbindungen der allgemeinen Formel (II) zu den Verbindungen der allgemeinen Formel (III) vorteilhafterweise kleiner als 2 : 1, vorzugsweise kleiner als 1,5 : 1, insbesondere ca. 1 : 1 ist.

4. Verfahren gemäß mindestens einem der Ansprüche 1 bis 3, **dadurch gekennzeichnet, dass** Mischungen von Verbindungen der allgemeinen Formel (III), mit R¹ = H und R¹ = C₁₋₃-Alkyl in Verhältnissen, in denen R¹ zu 0 bis 100 Gew.-%, vorzugsweise zu 0 bis 50 Gew.-%, insbesondere zu 10 bis 40 Gew.-% ein Wasserstoffatom ist, eingesetzt werden.

5. Verfahren gemäß Anspruch 4, in dem als Verbindungen der allgemeinen Formel (III) Mischungen von Verbindungen, bei denen R¹ ein Wasserstoffatom ist, mit solchen, bei denen R¹ eine lineare oder verzweigte Alkyl- oder Alkenylgruppe mit 1 bis 3 Kohlenstoffatomen ist, bevorzugt Methyl oder Ethyl, verwendet werden, die aus der Umsetzung von Glutaminsäure mit dem entsprechenden kurzkettigen Alkohol, vorzugsweise Methanol oder Ethanol, unter überkritischen Bedingungen hervorgegangen sind.

6. Verfahren gemäß mindestens einem der Ansprüche 1 bis 5, **dadurch gekennzeichnet, dass** ein immobilisiertes Enzym, beispielsweise Lipase aus *Candida rugosa, Pseudomonas sp., Thermomyces langosiosus,* Schweinepankreas, *Mucor miehei, Alcaligines sp.,* Cholesterol Esterase aus *Candida rugosa* oder Esterase aus Schweineleber, vorzugsweise Lipase B aus *Candida antarctica*, verwendet wird.
